# EUROPEAN PATENT APPLICATION

(11) **EP 3 916 733 A1**
(43) Date of publication of application: **01.12.2021**
(21) Application number: 21175797.6
(22) Date of filing: 25.05.2021
(51) Int. Cl.: G16H 20/70

(54) **METHOD, SYSTEM, AND COMPUTER PROGRAM PRODUCT FOR MATCHING STRESS-MANAGEMENT APPLICATION SOFTWARE TO THE NEEDS OF A USER**

(30) Priority: 28.05.2020 EP 20177026
(71) Applicant: Stichting IMEC Nederland, 6708 WH Wageningen (NL)
(72) Inventor: van Kraaij, Alex, 6532 CS Nijmegen (NL); Shiavone, Giuseppina, 4819 GG Breda (NL)
(74) Representative: Patent Department IMEC

(57) **Abstract**

A method for matching stress-management applications to the needs of a user is provided. The method comprises the steps of scoring the needs of the user on the basis of user input data in order to derive user scoring values, scoring the stress-management applications on the basis of specialist input data and/or additional input data relating to the stress-management applications in order to derive application scoring values with respect to each of the stress-management applications. Then computing a matching-score for each of the stress-management applications on the basis of the user scoring values and the application scoring values is computed.

## Description

### Technical field

The disclosure relates to a method for matching stress-management application software to the needs of a user, a system for matching stress-management application software to the needs of a user, and a computer program product comprising computer program code means adapted for matching stress-management application software to the needs of a user.

### Background

Generally, in times of an increasing number stress-management user applications, there is a growing need of an method, system, and computer program product for matching stress-management applications to the needs of a user in order to assist the user in choosing stress-management applications fitting his or her individual needs in a highly accurate and efficient manner.

EP 3 501 385 A1 relates to an electronic system for determining a subject's stress condition. A stress test unit is configured for receiving one or more features defining the subject and one or more physiological signals sensed from the subject when performing a relaxation and a stressful test task, for extracting normalization parameters of the physiological signals during the relaxation and stressful task and for identifying one or more physiological features that are responsive to stress. A storage unit is configured for storing a plurality of stress models generated based on a number of subjects, wherein each model has been trained with a different set of features. It is also used for storing the subject's features, normalization parameters and the stress-responsive physiological features. A stress detection unit is configured for selecting a stress model from the plurality of stored stress models based on the subject's features and the stress responsive physiological features and for estimating a specific stress condition based on the selected stress model. Subject's features, normalization parameters and one or more sensed physiological signals that apply to the selected stress model are stored, for providing a stress value representative of the subject's stress condition. However, said electronic system does not allow for matching stress-management applications to the needs of a user.

### Summary

Accordingly, there is an object to provide a method for matching stress-management applications to the needs of a user, a system for matching stress-management applications to the needs of a user, and a computer program product comprising computer program code means adapted for matching stress-management applications to the needs of a user.

According to a first aspect of the disclosure, a method for matching stress-management applications to the needs of a user is provided. The method comprises the steps of scoring the needs of the user on the basis of user input data in order to derive user scoring values, scoring the stress-management applications on the basis of specialist input data and/or additional input data relating to the stress-management applications in order to derive application scoring values with respect to each of the stress-management applications, and computing a matching-score for each of the stress-management applications on the basis of the user scoring values and the application scoring values by digital computing means. Advantageously, this allows a user to choose stress-management applications fitting his or her individual needs in a highly accurate and efficient manner.

According to a first example implementation form of the first aspect, the method further comprises the step of ranking the stress-management applications on the basis of the matching-score especially with respect to the needs of the user. Advantageously, for instance, simplicity can be increased, thereby also increasing efficiency.

According to a second example implementation form of the first aspect, the user input data is based on answers of the user to questions, preferably between 10 and 26 questions, more preferably between 16 and 20 questions, most preferably 18 questions, with respect to main aspects, especially six main aspects, in the context of acceptance and commitment of the user. Advantageously, for example, complexity can be reduced, which leads to an increased efficiency.

According to a further example implementation form of the first aspect, the main aspects in the context of acceptance and commitment of the user comprise at least one aspect of values, contact with present moment, committed action, self as context, cognitive defusion, acceptance, or any combination thereof. Advantageously, for instance, simplicity, and thus also efficiency, can further be increased.

According to a further example implementation form of the first aspect, the user input data is based on answers of the user to questions, preferably between 10 and 26 questions, more preferably between 16 and 20 questions, most preferably 18 questions, with respect to main aspects, especially six main aspects, in the context of cognitive behavior or lifestyle of the user. Advantageously, for example, complexity can further be reduced, thereby further increasing efficiency.

According to a further example implementation form of the first aspect, the main aspects in the context of lifestyle of the user comprise at least one aspect of nutrition, sports or exercise, social contacts, work or school, relaxation, sleep, or any combination thereof. Advantageously, for instance, efficiency can further be increased especially by increasing simplicity.

According to a further example implementation form of the first aspect, the method further comprises the step of visualizing the user scoring values in a hexagon with each of the six main aspects in a corner of the hexagon in the context of acceptance and commitment of the user. Advantageously, for example, efficiency can further be increased.

According to a further example implementation form of the first aspect, the method further comprises the step of visualizing the user scoring values in a hexagon with each of the six main aspects in a corner of the hexagon in the context of lifestyle of the user. Advantageously, for instance, visualization can further increase efficiency.

According to a further example implementation form of the first aspect, the method further comprises the step of visualizing the application scoring values with respect to each of the stress-management applications in a hexagon with each of six main aspects in a corner of the hexagon in the context of acceptance and commitment of the user. In addition to this or as an alternative, the method further comprises the step of visualizing the application scoring values with respect to each of the stress-management applications in a hexagon with each of six main aspects in a corner of the hexagon in the context of lifestyle of the user. Advantageously, for example, complexity can further be reduced, which leads to an increased efficiency.

According to a further example implementation form of the first aspect, the user input data is based on answers of the user to questions, preferably two questions, in the context of stress in general with respect to the user especially for deriving a general weight factor. Advantageously, for instance, accuracy can further be increased.

According to a further example implementation form of the first aspect, the user input data is based on answers of the user to questions, preferably three questions, in the context of demographics with respect to the user especially for dividing said answers into different categories, preferably five categories. Advantageously, for example, not only efficiency but also accuracy can further be increased.

According to a further example implementation form of the first aspect, deriving application scoring values with respect to each of the stress-management applications is based on machine-learning. Advantageously, for instance, both inaccuracies and inefficiencies can further be reduced.

According to a further example implementation form of the first aspect, for the machine-learning, the specialist input data is used as ground truth. Advantageously, for example, accuracy can further be increased.

According to a second aspect of the disclosure, a system for matching stress-management applications to the needs of a user is provided. The system comprises at least one input unit and one processing unit. In this context, the input unit is configured to receive user input data. Additionally, the input unit is further configured to receive specialist input data and/or additional input data relating to the stress-management applications. In further addition to this, the processing unit is configured to score the needs of the user on the basis of user input data in order to derive user scoring values, to score the stress-management applications on the basis of specialist input data and/or additional input data relating to the stress-management applications in order to derive application scoring values with respect to each of the stress-management applications, and to compute a matching-score for each of the stress-management applications on the basis of the user scoring values and the application scoring values. Advantageously, this allows a user to choose stress-management applications fitting his or her individual needs in a highly accurate and efficient manner.

According to a third aspect of the disclosure, a computer program product comprising computer program code means is provided. Said computer program comprising computer program code means is adapted for matching stress-management applications to the needs of a user according to the steps of the method or any of the preferred implementation forms thereof when said program is run on a computer or any electronic system. Advantageously, this allows a user to choose stress-management applications fitting his or her individual needs in a highly accurate and efficient manner.

### Brief description of the drawings

Exemplary embodiments of the disclosure are now further explained with respect to the drawings by way of example only, and not for limitation. In the drawings:
- Fig. 1: shows a flow chart of an exemplary embodiment of a method according to the present disclosure;
- Fig. 2: shows an exemplary embodiment of a system according to the present disclosure;
- Fig. 3: shows an example flow chart of a method according to the present disclosure;
- Fig. 4: shows an example hexagonal comparison grid chart with respect to acceptance and commitment of the user;
- Fig. 5: shows an example hexagonal comparison grid chart with respect to lifestyle of the user;
- Fig. 6: shows an example overview of scoring user input data;
- Fig. 7: shows an example overview of scoring stress-management applications; and
- Fig. 8: shows an example table for computing a matching score.

### Description of illustrative embodiments

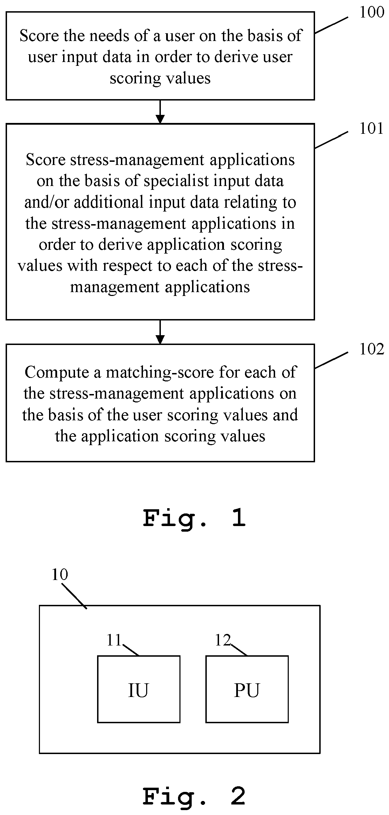

Firstly, Fig. 1 shows a flow chart of an embodiment of a method for matching stress-management applications to the needs of a user. In a first step 100, the needs of the user are scored on the basis of user input data in order to derive user scoring values. The input data and the scoring values can be transported and/or can be represented by electronic signals. Then, in a second step 101, the stress-management applications are scored on the basis of specialist input data and/or additional input data relating to the stress-management applications in order to derive application scoring values with respect to each of the stress-management applications. Furthermore, in a third step 102, a matching-score is computed for each of the stress-management applications on the basis of the user scoring values and the application scoring values by mean of digital computing means such as a digital data processor. The data can be stored by electronic memory means such as SRAM or DRAM.

It might be advantageous if the method further comprises the step of ranking the stress-management applications on the basis of the matching-score especially with respect to the needs of the user.

In addition to this or as an alternative, the user input data may especially be based on answers of the user to questions, preferably between 10 and 26 questions, more preferably between 16 and 20 questions, for example 18 questions, with respect to main aspects, especially six main aspects, in the context of acceptance and commitment of the user.

It is noted that the main aspects in the context of acceptance and commitment of the user may preferably comprise at least one aspect of values, contact with present moment, committed action, self as context, cognitive defusion, acceptance, or any combination thereof. It is further noted that it might be particularly advantageous if the user input data is based on answers of the user to questions, preferably between 10 and 26 questions, more preferably between 16 and 20 questions, most preferably 18 questions, with respect to main aspects, especially six main aspects, in the context of lifestyle of the user.

Furthermore, the main aspects in the context of lifestyle of the user may preferably comprise at least one aspect of nutrition, sports or exercise, social contacts, work or school, relaxation, sleep, or any combination thereof. Moreover, the method may further comprise the step of visualizing the user scoring values in a hexagon with each of the six main aspects in a corner of the hexagon in the context of acceptance and commitment of the user. It is further noted that the method may additionally or alternatively comprise the step of visualizing the user scoring values in a hexagon with each of the six main aspects in a corner of the hexagon in the context of lifestyle of the user.

It might be advantageous if the method further comprises the step of visualizing the application scoring values with respect to each of the stress-management applications in a hexagon with each of six main aspects in a corner of the hexagon in the context of acceptance and commitment of the user. It is noted that the main aspects in the context of acceptance and commitment of the user may preferably comprise at least one aspect of values, contact with present moment, committed action, self as context, cognitive defusion, acceptance, or any combination thereof.

In addition to this or as an alternative, the method may further comprise the step of visualizing the application scoring values with respect to each of the stress-management applications in a hexagon with each of six main aspects in a corner of the hexagon in the context of lifestyle of the user. In this context, it is further noted that the main aspects in the context of lifestyle of the user may preferably comprise at least one aspect of nutrition, sports or exercise, social contacts, work or school, relaxation, sleep, or any combination thereof.

With respect to the user input data, it is noted that the user input data may preferably be based on answers of the user to questions, for example two questions, in the context of stress in general with respect to the user especially for deriving a general weight factor. In addition to this or as an alternative, the user input data may preferably be based on answers of the user to questions, for example three questions, in the context of demographics with respect to the user especially for dividing said answers into different categories, for example five categories.

It is further noted that it might be advantageous if deriving application scoring values with respect to each of the stress-management applications is based on machine-learning. In this context, it is further noted that for the machine-learning, the specialist input data may preferably be used as ground truth.

With respect to Fig. 2, a block diagram of an example embodiment of a system 10 for matching stress-management applications to the needs of a user is shown. According to Fig. 2, the system 10 comprises an input unit 11 and a processing unit 12. The processing unit 12 may be a digital data processor or a network of distributed digital data processors. In this context, the input unit 11 is configured to receive user input data. According to an example embodiment, the input unit 11 may be one or a plurality of input units connected to the system and further configured to receive specialist input data and/or additional input data relating to the stress-management applications.

In an example embodiment, the processing unit 12 is configured to score the needs of the user on the basis of user input data in order to derive user scoring values, to score the stress-management applications on the basis of specialist input data and/or additional input data relating to the stress-management applications in order to derive application scoring values with respect to each of the stress-management applications, and to compute a matching-score for each of the stress-management applications on the basis of the user scoring values and the application scoring values.

It is noted that it might be advantageous if the processing unit 12 is further configured to rank the stress-management applications on the basis of the matching-score especially with respect to the needs of the user. Furthermore, the user input data may especially be based on answers of the user to questions, preferably between 10 and 26 questions, more preferably between 16 and 20 questions, for example 18 questions, with respect to main aspects, especially six main aspects, in the context of acceptance and commitment of the user. Moreover, the main aspects in the context of acceptance and commitment of the user may preferably comprise at least one aspect of values, contact with present moment, committed action, self as context, cognitive defusion, acceptance, or any combination thereof.

It is further noted that it might be advantageous if the user input data is based on answers of the user to questions, preferably between 10 and 26 questions, more preferably between 16 and 20 questions for example 18 questions, with respect to main aspects, especially six main aspects, in the context of lifestyle of the user. Furthermore, the main aspects in the context of lifestyle of the user may preferably comprise at least one aspect of nutrition, sports or exercise, social contacts, work or school, relaxation, sleep, or any combination thereof.

It might be advantageous if especially with the aid of a display unit, the processing unit 12 is further configured to visualize the user scoring values in a hexagon with each of the six main aspects in a corner of the hexagon in the context of acceptance and commitment of the user. In addition to this or as an alternative, especially with the aid of a display unit or the above-mentioned display unit, the processing unit 12 may further be configured to visualize the user scoring values in a hexagon with each of the six main aspects in a corner of the hexagon in the context of lifestyle of the user.

Furthermore, especially with the aid of a display unit or the above-mentioned display unit, the processing unit 12 may further be configured to visualize the application scoring values with respect to each of the stress-management applications in a hexagon with each of six main aspects in a corner of the hexagon in the context of acceptance and commitment of the user. It is noted that the main aspects in the context of acceptance and commitment of the user may preferably comprise at least one aspect of values, contact with present moment, committed action, self as context, cognitive defusion, acceptance, or any combination thereof.

Moreover, especially with the aid of a display unit or the above-mentioned display unit, the processing unit 12 may further be configured to visualize the application scoring values with respect to each of the stress-management applications in a hexagon with each of six main aspects in a corner of the hexagon in the context of lifestyle of the user. It is further noted that the main aspects in the context of lifestyle of the user may preferably comprise at least one aspect of nutrition, sports or exercise, social contacts, work or school, relaxation, sleep, or any combination thereof.

Furthermore, the user input data may especially be based on answers of the user to questions, for example two questions, in the context of stress in general with respect to the user especially for deriving a general weight factor. In addition to this or as an alternative, the user input data may especially be based on answers of the user to questions, for example three questions, in the context of demographics with respect to the user especially for dividing said answers into different categories, preferably five categories.

It is noted that it might be advantageous if deriving application scoring values with respect to each of the stress-management applications is based on machine-learning. In this context, the processing unit 12 may especially provide machine-learning capabilities. Furthermore, it is noted that for the machine-learning, the specialist input data may preferably be used as ground truth.

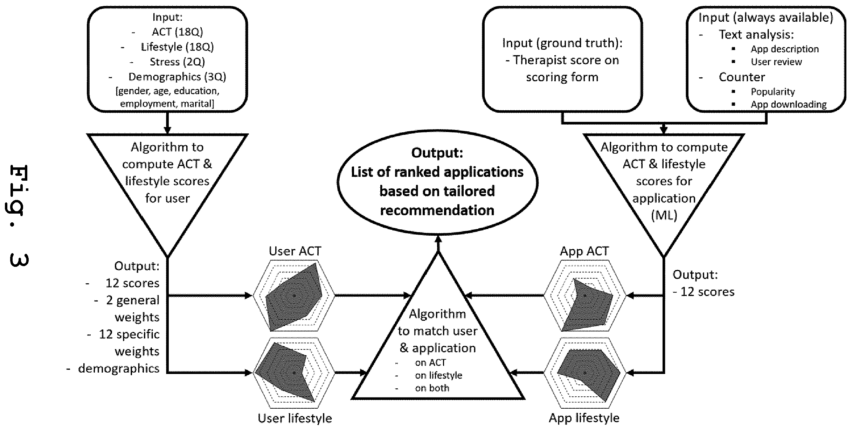

Now, with respect to Fig. 3, a more detailed kind of flow chart of an exemplary embodiment of the inventive method is shown. In this context, it is noted that with respect to the above-mentioned acceptance and commitment or lifestyle, respectively, ACT (acceptance and commitment therapy) and lifestyle will be used in the following. The ACT especially concerns six main aspects, lifestyle is also subdivided into six aspects in this matter.

Furthermore, a potential user will first fill in for example 41 questions, of which for example 18 about the ACT aspects, for example 18 about the lifestyle aspects, two on stress in general and three on demographics (gender, age and education level). From the 18 questions on the ACT aspects, six are especially derived of an existing ACT weekly diary, six are especially two-choice questions and six are especially grading questions (1-10).

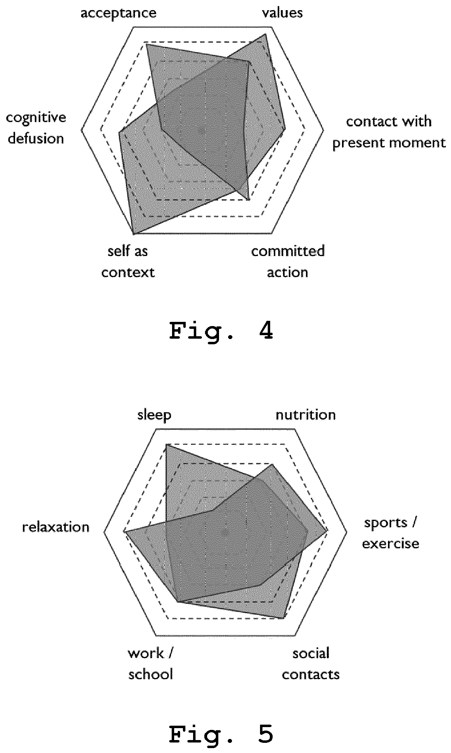

Although there is no lifestyle weekly diary, the questions related to the lifestyle components are preferably be inspired by the ACT questions. The two general questions on stress are especially derived from the existing ACT weekly diary. Running the inventive method over the filled-out questionnaires, especially gives a score for every aspect of the ACT and lifestyle, a weight factor for every aspect, two general weight factors and some demographic details. The scores are visualized in a hexagon with every aspect in a corner. The hexagons for the ACT and lifestyle aspects including potential scores are shown in Fig. 4 and Fig. 5.

The same comparison hexagons are especially made for the stress management applications, only with a different input. For the calculation of these scores machine-learning capabilities may preferably be used. Different stress-management applications may especially be scored by a specialist such as a psychotherapist preferably with a provided scoring form. These results are especially the ground truth.

Furthermore, especially based on at least one of text analysis of the description of the respective stress-management application, user reviews being especially available on a server providing the respective stress-management application such as an application store in the internet, counting popularity with respect to the respective stress-management application, counting application downloading, the inventive method or its machine-learning capabilities may especially be trained preferably to predict the specialist scores.

As output a score for every aspect of the ACT and lifestyle may especially be calculated, and the hexagon comparison grids are preferably generated. Moreover, the scores of the stress-management applications may be compared with the specific user needs to generate a ranked list of applications, tailored to the user's need. As input for the stress-management applications, the scores are preferably used. As input for the user needs, the scores are especially first weighted and categorized with help of the weighting factors and demographics. The method can rank the applications based on only ACT aspects, only lifestyle aspects or both aspects. It is further noted that the steps in the inventive method can basically be divided into three major steps: The user scoring step, the application scoring step, and the application-user matching step. Below the three major steps will be explained in more detail.

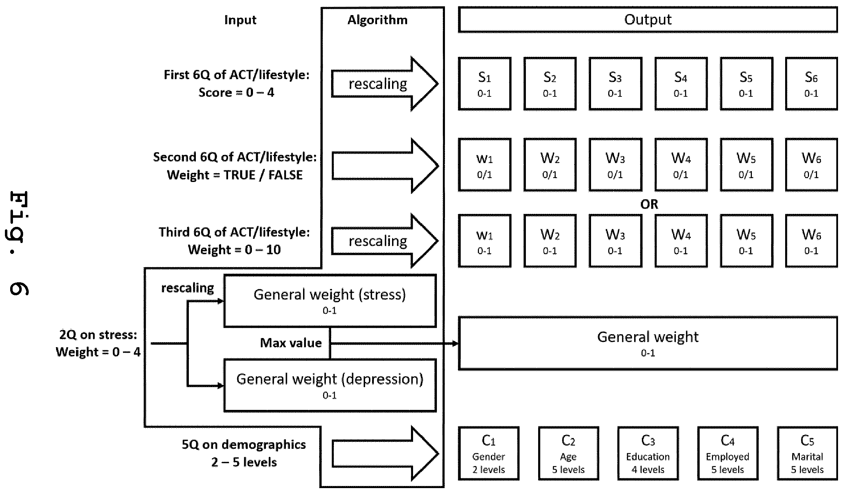

With respect to the user scoring step, in accordance with Fig. 6, this step especially uses the questionnaires filled out by the user as input and has multiple outputs for the ACT aspects, lifestyle aspects and in general. In Fig. 6, it is shown how the output is computed from the input. The first six questions on the ACT aspects, that are derived of the corresponding ACT weekly diary, are rescaled resulting in a score ranging from 0 to 1 for every aspect.

The same is done for the lifestyle aspects. The output shown in Fig. 6 is referring to only ACT or lifestyle, so in total there are 12 scores in the shown example. The second six questions, referring to the questions for support, do not need any transformation and the responses can be used straight as weight factors. However, these weight factors are optional. The second option that can be used as weight factor for the aspect scores is based on the rating questions for the ACT and lifestyle aspects.

Again, Fig. 6 shows the output of only ACT or lifestyle, so the number of weight factors is doubled. After choosing between the optional weight factors, this results in 12 aspect-specific weight factors in total. Based on the two general questions on stress and depression, one general weight factor is derived. This is especially done by first rescaling both the question responses and then choosing the maximum value, since a high score on one of the questions is already affecting the importance of the other scores.

This general weight factor is especially the same for the ACT and lifestyle, so there is only one. Finally, the responses on the demographic questions are especially stored into five categories as shown in Fig. 6. These outputs will preferably be used as inputs on the user needs for the matching step.

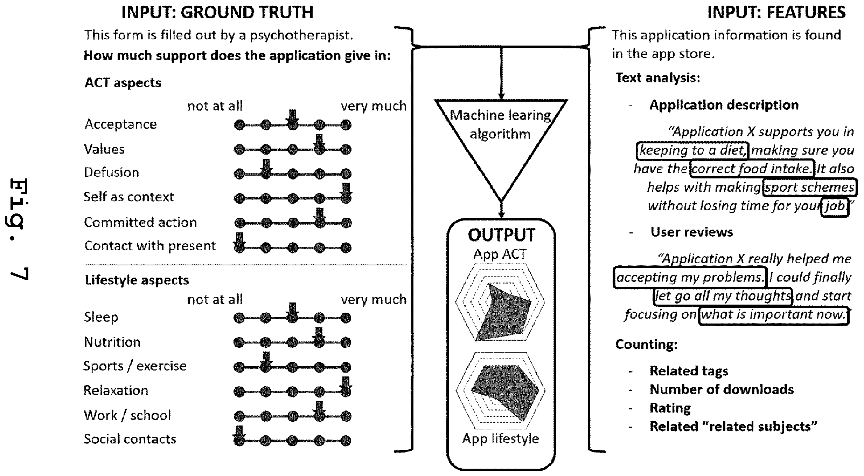

Now, with respect to the application scoring step, in accordance with Fig. 7, especially a small set of specialists will be asked to rate several stress-management applications exemplarily from the application store in the internet. This will be preferably be done with a rating form shown on the left side of Fig. 7. The six aspects of both the ACT and lifestyle will be rated for every stress management application. This will especially be used as ground truth preferably for the machine-learning capabilities of the inventive method.

This machine-learning capabilities may especially predict these scores based on the features derived exemplarily from the application store. These may preferably be text features such as key words in the application description and user reviews on the application store and these are numeric features such as the number of related tags, downloads, ratings and related topics, exemplarily suggested by the application store, related to an aspect.

The scores on the ACT and lifestyle aspects, as output of this step, will preferably be used as input for the user-application matching step. With respect to said matching step, the scores and weights derived from the user scoring step are especially combined with the scores derived from the application scoring step preferably to compute a matching-score for every application, which is especially used to rank the applications based on their fit to the user's needs.

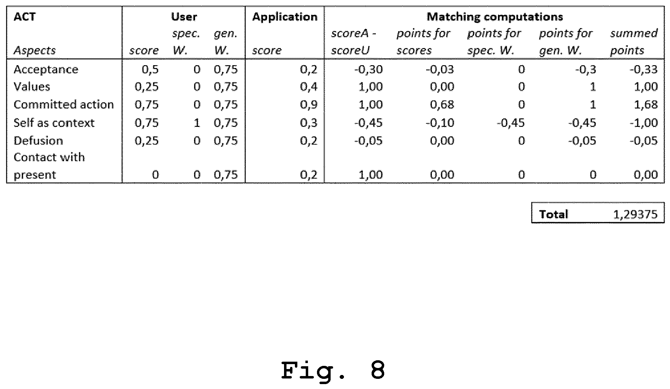

Fig. 8 shows an example of comparing an application to a user based on only the ACT aspects. The computations for only the lifestyle aspects are completely similar and for comparing on both the ACT and lifestyle aspects, the total scores are especially to be summed up. Below all the variables in the "matching computations" section are explained. The "user" section and "application" section are the outputs of the previous discussed major steps and are used for the calculations of the remaining variables.
- 'scoreA - scoreU': In this column it is first checked if the application score for an aspect is higher or equal to the user's score for that aspect. If so, it will show a 1. If not, it will calculate the difference and show what the application is short. This variable is only an intermediate variable and not summed up in the final points.
- "points for scores": In this column it is first checked if the user's score is 0.5 or higher, meaning there is some need for support on this aspect. If so, it multiplies the user score by the application score by the 'scoreA - scoreU' to include the severity of the user's need, the assessed contribution of the application and how well they match of the aspect. This is the first part of the points summed in the last column.
- "points for spec. W.': In this column it is first checked if the specific weight factor is equal or higher than 0.5. Now it shows the specific weight factor based on the binomial questions, but this 'IF'-module also works for the rating questions. If the specific weight factor is equal or higher than 0.5, the 'scoreA - scoreU' is shown. If not, it will be 0, since there is then no need based on the specific weight factor for this application. These points are also included in the summation in the last column.
- "points for gen. W.': The computations in this column are similar to the computations for "points for spec. W.', only now it is first checked if the general weight factor is equal or higher than 0.5 and then checked if the user score is higher than 0. If both are true, the 'scoreA - scoreU' is shown. If one of them is not true, this means there is no need for the application for this aspect or in general and so the points are set to 0.
- "summed points': In this column, the points are summed up for all the aspects. Finally, the total matching-score is calculated and represented in the bottom right. The higher this score, the better the application matches the user's needs. Based on this score, the application will be ranked.

Finally, it is noted that in the user-application matching step described above, the demographics categories are not yet used. These categories could also serve as Booleans to include the ' scoreA - scoreU' for an aspect. Based on research, it could for example be found that acceptance has a significant higher impact on chronic stress for male than for female. If a user is male, the 'scoreA - scoreU' for acceptance could then be included in the summed points, while for female it stays 0. This could be done for all other demographics as well.

While various embodiments of the present invention have been described above, it should be understood that they have been presented by way of example only, and not limitation. Numerous changes to the disclosed embodiments can be made in accordance with the disclosure herein without departing from the spirit or scope of the invention. Thus, the breadth and scope of the present invention should not be limited by any of the above-described embodiments. Rather, the scope of the invention should be defined in accordance with the following claims and their equivalents.

Although the invention has been illustrated and described with respect to one or more implementations, equivalent alterations and modifications will occur to others skilled in the art upon the reading and understanding of this specification and the annexed drawings. In addition, while a particular feature of the invention may have been disclosed with respect to only one of several implementations, such feature may be combined with one or more other features of the other implementations as may be desired and advantageous for any given or particular application.

## Claims

1. A computer-implemented method for matching stress-management software applications to the needs of a user, the method comprising the steps of:
receiving, by an input unit (11), user input data associated to a plurality of user specific questions,
computing, by a processing unit (12), scores values (100) for the needs of the user on the basis of the user input data in order to derive user scoring values,
receiving, by an input unit (11), specialist input data and/or additional input data, wherein the specialist input data comprises answers corresponding to a plurality of stress-management applications, and wherein the additional input data comprises features derived from the stress-management applications,
computing, by the processing unit (12), score values (101) for the stress-management applications on the basis of the specialist input data and/or the additional input data in order to derive application scoring values with respect to each of the stress-management applications, and
computing (102), by the processing unit (12), a matching-score for each of the stress-management applications on the basis of the user scoring values and the application scoring values.

2. The method according to claim 1,
wherein the method further comprises the step of computationally ranking, by the processing unit (12), the stress-management applications on the basis of the matching-score especially with respect to the needs of the user.

3. The method according to claim 1 or 2,
wherein the user input data is based on answers of the user to questions, with respect to main aspects, in the context of acceptance and commitment of the user, wherein main aspects in the context of acceptance and commitment of the user comprise at least one aspect of values, contact with present moment, committed action, self as context, cognitive defusion, acceptance, or any combination thereof.

4. The method according to any of the claims 1 to 3,
wherein the user input data is based on answers of the user to the questions, with respect to main aspects, in the context of lifestyle of the user, wherein the main aspects in the context of lifestyle of the user comprise at least one aspect of nutrition, sports or exercise, social contacts, work or school, relaxation, sleep, or any combination thereof.

5. The method according to claim 3 or 4,
wherein the method further comprises the step of providing information about the user scoring values with each of the main aspects in the context of acceptance and commitment of the user, wherein the information comprises at least a score and a weight factor for each of the main aspects in the context of acceptance and commitment of the user.

6. The method according to claim 4,
wherein the method further comprises the step of providing information about the user scoring values with each of the main aspects in the context of lifestyle of the user, wherein the information comprises at least a score and a weight factor for each of the main aspects in the context of lifestyle of the user.

7. The method according to any of the claims 1 to 6,
wherein the user input data is based on answers of the user to questions, in the context of perceived stress in general with respect to the user especially for deriving a general weight factor.

8. The method according to any of the claims 1 to 7,
wherein deriving application scoring values with respect to each of the stress-management applications is based on machine-learning computing techniques, wherein for the machine-learning computing, the specialist input data is used as ground truth.

9. The method according to any of the claims 1 to 8,
wherein the specialist input data is based on answers of a specialist or therapist to questions, with respect to main aspects, in the context of acceptance and commitment of the user and/or the lifestyle of the user, and
wherein the method further comprises the step of training machine-learning capabilities of the processing unit using the specialist input data, wherein the machine-learning capabilities predict the score values.

10. The method according to claim 9,
wherein the method further comprises the step of providing information about the application scoring values with each of the main aspects in the context of the acceptance and commitment of the user, wherein the information comprises at least a score for each of the main aspects in the context of acceptance and commitment of the user.

11. The method according to claim 9 or 10,
wherein the method further comprises the step of providing information about the application scoring values with each of the main aspects in the context of the lifestyle of the user, wherein the information comprises at least a score for each of the main aspects in the context of the lifestyle of the user.

12. The method according to any of the claims 1 to 11,
wherein the method of computing the matching-score further comprises the step of comparing the user scoring values with the application scoring values to generate:
points for scores associated with the user scoring values and the application scoring values, and
points for weights associated with the user scoring values.

13. The method according to claim 12,
wherein the method further comprises the step of summing the points generated for the scores and the weights to compute the matching-score for each of the stress-management applications.

14. A system (10) for matching stress-management software applications to the needs of a user, the system comprising at least one of:
an input unit (11), and
a processing unit (12),
wherein the input unit (11) is configured to receive user input data associated to a plurality of user specific questions,
wherein the input unit (11) is further configured to receive specialist input data and/or additional input data relating to the stress-management applications, wherein the specialist input data comprises answers corresponding to a plurality of stress-management applications, and wherein the additional input data comprises features derived from the stress-management applications, and
wherein the processing unit (12) is configured to compute score values for the needs of the user on the basis of user input data in order to derive user scoring values, to compute score values for the stress-management applications on the basis of specialist input data and/or additional input data in order to derive application scoring values with respect to each of the stress-management applications, and to compute a matching-score for each of the stress-management applications on the basis of the user scoring values and the application scoring values.

15. A computer program product comprising computer program code means adapted for matching stress-management software applications to the needs of a user according to all steps of the method of any of the claims 1 to 13 when said program is run on the system according to claim 14.
